# DEMANDE DE BREVET EUROPEEN

(11) **EP 1 847 295 A2**
(43) Date de publication de la demande: **24.10.2007**
(21) Numéro de dépôt: 07105943.0
(22) Date de dépôt: 11.04.2007
(51) Int. Cl.: A61Q 5/06, A61K 8/40, A61K 8/49

(54) **Composition de coloration comprenant un composé fluorescent soluble, au moins un monomère électrophile et au moins un solvant organique liquide**

(30) Priorité: 13.04.2006 FR 0603321
(71) Demandeur: L'Oréal, 75008 Paris (FR)
(72) Inventeur: Chaisy, Maryse, 95190 Goussainville (FR); Gourlaouen, Luc, 92600 Asnieres (FR)
(74) Mandataire: Fevrier, Murielle Françoise E.

(57) **Abrégé**

La présente invention qui a pour objet une composition de coloration des fibres kératiniques comprenant, au moins un monomère électrophile, au moins un composé fluorescent soluble et au moins un solvant organique liquide

La composition conforme à la présente invention permet en particulier d'améliorer la visibilité de la coloration sur les fibres kératiniques foncées tout en présentant une résistance améliorée aux agents extérieurs.

## Description

La présente invention a pour objet une composition de coloration des fibres kératiniques, en particulier les cheveux, comprenant au moins un composé fluorescent soluble, au moins un monomère électrophile et au moins un solvant organique liquide.

Il est connu de teindre les fibres kératiniques, notamment humaines, par une coloration directe. Le procédé classiquement utilisé en coloration directe consiste à appliquer sur les fibres kératiniques des colorants directs qui sont des molécules colorées et colorantes ayant une affinité pour les fibres, à les laisser diffuser, puis à rincer les fibres.

Les colorants directs qui sont classiquement utilisés sont par exemple des colorants du type nitrés benzéniques, des colorants anthraquinoniques, des nitropyridines, des colorants du type azoïque, xanthénique, acridinique, azinique ou triarylméthane.

Les colorations qui résultent de l'utilisation de colorants directs sont des colorations temporaires ou semi-permanentes car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration.

Par ailleurs, la coloration des fibres kératiniques à partir de colorants directs classiques ne permet pas d'éclaircir de façon notable les fibres kératiniques.

L'éclaircissement de la couleur de fibres kératiniques constitue une demande importante.

Classiquement, pour obtenir une coloration plus claire on met en oeuvre un procédé de décoloration chimique. Ce procédé consiste à décolorer les mélanines contenues dans les fibres par un système oxydant, généralement constitué par du peroxyde d'hydrogène associé ou non à des persels, le plus souvent en milieu alcalin.

Ce système de décoloration présente l'inconvénient de dégrader les fibres et d'altérer leurs propriétés cosmétiques. Les cheveux ont en effet tendance à devenir rêches, plus difficilement démêlables et plus fragiles. Ainsi l'éclaircissement des cheveux, s'accompagne d'une diminution non négligeable des qualités mécaniques de la chevelure.

Enfin, l'éclaircissement ou la décoloration de fibres kératiniques à partir d'agent oxydant est incompatible avec les traitements de modification de la forme desdites fibres particulièrement dans les traitements de défrisage.

Une autre technique d'éclaircissement consiste à appliquer sur les cheveux foncés des colorants directs fluorescents. Cette technique décrite notamment dans le document FR 2830189 permet de respecter la qualité de la fibre kératinique lors du traitement mais les colorants fluorescents employés ne présente pas une bonne résistance aux shampoings.

Par ailleurs, il est connu de la demande de brevet FR 2 833 489 des compositions de traitement des cheveux à partir de compositions comprenant des monomères électrophiles. Une telle composition permet d'obtenir des cheveux parfaitement gainés et non gras.

Le but de la présente invention est de fournir de nouvelles compositions pour la coloration des fibres kératiniques notamment les cheveux, qui permettent d'obtenir des colorations visibles sur cheveux foncés sans qu'il soit nécessaire d'éclaircir ou de décolorer les fibres et qui présentent une bonne résistance aux agents extérieurs.

Ce but est atteint avec la présente invention qui a pour objet une composition de coloration comprenant, au moins un monomère électrophile, au moins un composé fluorescent soluble et au moins un solvant organique liquide.

La composition conforme à la présente invention permet d'améliorer la visibilité de la coloration notamment sur cheveux foncés. En particulier, on obtient dans le cas des fibres kératiniques foncées une coloration très visible sans qu'il soit nécessaire d'éclaircir ou de décolorer les fibres kératiniques et par conséquent sans dégradation physique des fibres kératiniques. Cette composition permet en particulier d'obtenir des effets d'éclaircissement optique sur cheveux foncés, notamment des cheveux qui présentent une hauteur de ton inférieure ou égale à 6.

Elle permet aussi par le choix du composé fluorescent d'obtenir des colorations avec des effets spéciaux sous l'effet de lumière riche en rayonnement UV comme les éclairages utilisés dans les boites de nuit par exemple.

De plus, cette coloration présente une bonne résistance aux diverses agressions que peuvent subir les cheveux telles que les shampooings, les frottements, la lumière, les intempéries, la sueur et les déformations permanentes. Ces propriétés sont particulièrement remarquables en ce qui concerne la résistance de la coloration vis à vis des shampooings.

La présente invention a aussi pour objet un procédé de coloration des fibres kératiniques qui comprend l'application sur les fibres kératiniques de la composition de l'invention ainsi que l'utilisation de la composition de l'invention pour la coloration des fibres kératiniques, en particulier les cheveux.

Un autre objet de l'invention concerne un kit comprenant d'une part une composition comprenant le composé fluorescent tel que défini précédemment et d'autre part un monomère électrophile, le solvant organique liquide pouvant être dans l'une et/ou l'autre des compositions.

Dans le cadre de l'invention, on entend par composé fluorescent soluble, un composé qui présente une solubilité dans le milieu supérieure à 0,0001 % à 20 °C, préférablement supérieure à 0,01 %.

On entend par ailleurs par composé fluorescent des colorants fluorescents et des azurants optiques.

Les colorants fluorescents sont des composés fluorescents qui absorbent dans le rayonnement visible généralement entre 400 et 800 nm et qui sont capables de réémettre de la lumière dans le domaine du visible à une longueur d'onde supérieure. Par définition, ces colorants sont des espèces colorées puisqu'elles absorbent de la lumière visible.

Selon un mode de réalisation particulier, les colorants fluorescents utiles dans le cadre de l'invention réémettent de la lumière fluorescente orangée. Ils présentent notamment une longueur d'onde maximum de ré-émission comprise entre comprise entre 500 et 700 nm.

A titre d'exemple de colorants fluorescents, on peut citer les composés connus de la technique, décrits par exemple dans les ouvrages suivants : Ullmann's Encyclopedia of Industrial Chemistry Release 2004, 7th edition, chapitre « Fluorescent Dyes ».

Les azurants optiques utiles dans la présente invention aussi connus sous le nom de "brighteners", ou "fluorescent brighteners", ou "fluorescent brightening agents" ou "FWA", ou "fluorescent whitening agents", ou "whiteners", ou encore "fluorescent whiteners" en terminologie anglo-saxone, sont des composés transparents incolores car il n'absorbent pas dans la lumière visible, mais uniquement dans les Ultra Violets (longueurs d'onde allant de 200 à 400 nanomètres), et transforment l'énergie absorbée en lumière fluorescente de plus grande longueur d'onde émise dans la partie visible du spectre ; en général dans le bleu et/ou le vert, c'est-à-dire dans des longueurs d'onde allant de 400 à 550 nanomètres.

Les azurants optiques sont connus de la technique. Ils sont par exemple décrits dans Ullmann's Encyclopedia of Industrial Chemistry (2002) "Optical Brighteners" et Kirk-Othmer Encyclopedia of Chemical Technology (1995); "Fluorescent Whitening Agents".

Les colorants fluorescents qui peuvent être utilisés dans le cadre de l'invention sont des composés connus de la technique. Ils sont par exemple décrits dans le document FR 2 830 189. A titre d'exemples de colorants fluorescents, on peut notamment citer :
- le Photosensitizing Dye NK-557 commercialisé par la société UBICHEM qui présente la structure suivante : 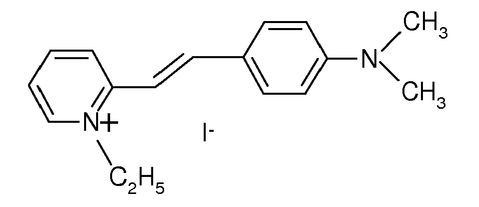 iodure de 2-[2-(4-diméthylamino)phényl éthényl]-1 éthyl-pyridinium ;
- le Jaune Brilliant B6GL commercialisé par la société SANDOZ et de structure suivante : 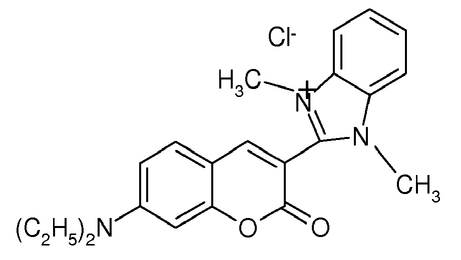
- le Basic Yellow 2, ou Auraminoe O commercialisé par les sociétés PROLABO, ALDRICH ou CARLO ERBA et de structure suivante 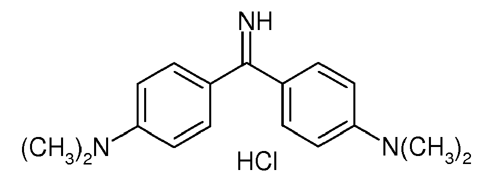 monochlorhydrate de 4,4'-(imidocarbonyl)bis(N,N-diméthylaniline)

A titre d'azurants optiques et de colorants fluorescents utiles dans la présente invention on peut aussi citer :
- Les naphthalimides, par exemple le composé suivant 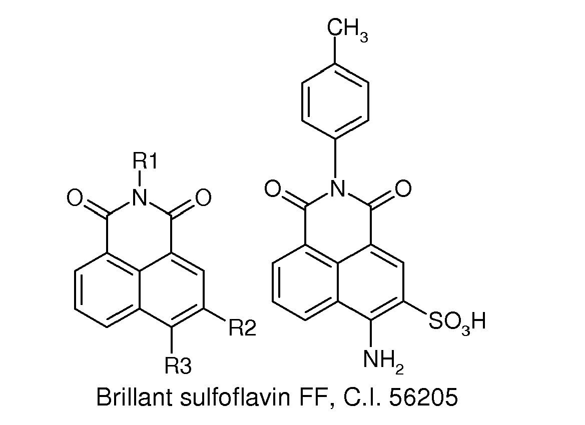 R1, R2, R3, indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6 ; un groupement pipéridinosulfonyle, un groupement pyrrolidino, un groupement alkyle(C1-C6)halogénoalkyle(C1-C6)amino ; un groupement benzoylalkyle(C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que N, S ou O ; les substituants R1 , R2, R3 peuvent former avec les atomes de carbone auxquels ils sont rattachés un cycle aromatique ou non, en C6-C30 ou hétérocyclique comportant au total de 5 à 30 chaînons et de 1 à 5 hétéroatomes ; ces cycles étant condensés ou non, insérant ou non un groupement carbonyle, et étant substitués ou non par un ou plusieurs groupements choisis parmi les groupements alkyle en C1-C4, alcoxy(C1-C4)alkyl(C1-C4), amino, dialkyl(C1-C4)amino, halogène, phényle, carboxy, trialkyl(C1-C4)ammonioalkyl(C1-C4).
- Les dérivés de coumarines tels que les composés correspondants aux formules suivantes 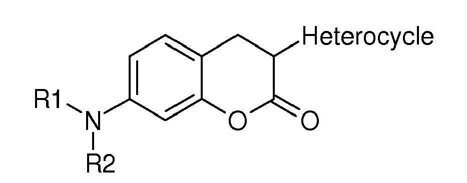 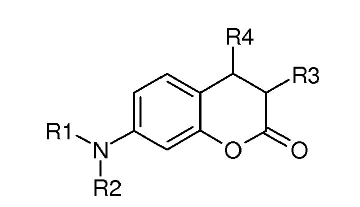
dans laquelle l'hétérocycle est choisi parmi les furane, tiophène, 2H-pyrrole, 2-pyrroline, pyrrolidine, 1,3-dioxolane, oxazole, thiazole, Imidazole, 2-imidazoline, Imidazoline, pyrazole, 2-pyrazoline, pyrazolidine, isoxazole, isothiazole, 1,2,3-oxadiazole, 1,2,3-triazole, 1,3,4-thiadiazole, 2H-pyran, 4H-pyran, pyridine, piperidine, 1,4-dioxane, morpholine, 1,4-dithiane, thiomorpholine, pyradizine, pyrimidine, pyrazine, piperazine, 1,3,5-Triazine, 1,3,5-trithiane, indolizine, indole, isoindole, 3H-indole, indoline, benzo[b]furan, benzo[b]thiophene, 1 H-indazole, benzimidazole, benzothiazole, purine, 4H-quinolizine, quinoline, isoquinoline, cinnoline, phtalazine, quinazoline, quinoxaline, 1,8-naphtyridine, pteridine, quinuclidine, carbazole, acridine, phenazine, phenothiazine, phenoxazine, indene, naphtalene, azulene, fluorene, anthracene, norbornane, adamantane, et R1, R2, R3, R4 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6 ; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène, ce radical alkyle pouvant être interrompu par un hétéroatome, tel qu'un atome d'azote, de soufre ou d'oxygène ; les substituants R1, R2, R3 et R4 peuvent former avec les atomes de carbone auxquels ils sont rattachés un cycle aromatique ou non, en C6-C30 ou hétérocyclique comportant au total de 5 à 30 chaînons et de 1 à 5 hétéroatomes ; ces cycles étant condensés ou non, insérant ou non un groupement carbonyle, et étant substitués ou non par un ou plusieurs groupements choisis parmi les groupements alkyle en C1-C4, alcoxy(C1-C4)alkyl(C1-C4), amino, dialkyl(C1-C4)amino, halogène, phényle, carboxy, trialkyl(C1-C4)ammonioalkyl(C1-C4) ; deux des substituants R3 , R4 peuvent former avec les atomes de carbone auxquels ils sont rattachés un noyau aromatique en C6-C30 ou hétérocyclique comportant au total de 5 à 30 chaînons et de 1 à 5 hétéroatomes ; ce cycle étant condensés ou non,ce cycle et l'éventuel cycle condensé étant substitués ou non par au moins un groupement choisi parmi les groupements alkyle en C1-C4, alcoxy(C1-C4)alkyl(C1-C4), amino, dialkyl(C1-C4)amino, halogène, phényle, carboxy, trialkyl(C1-C4)ammonioalkyl(C1-C4).

A titre d'exemple, on peut citer 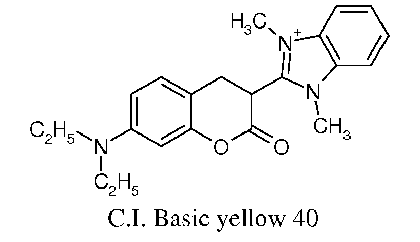
- Les dérivés de xanthènes tels que : 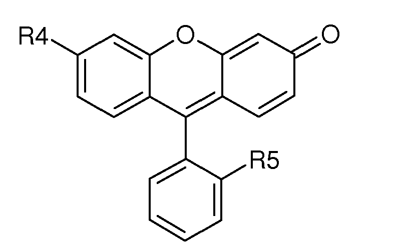 avec R4 et R5 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6 ; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène.
- Les rhodamines telles que 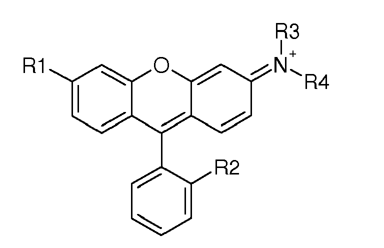
dans laquelle R1, R2, R3 et R4 sont tels que définis ci-dessus. A titre d'exemple, on peut citer 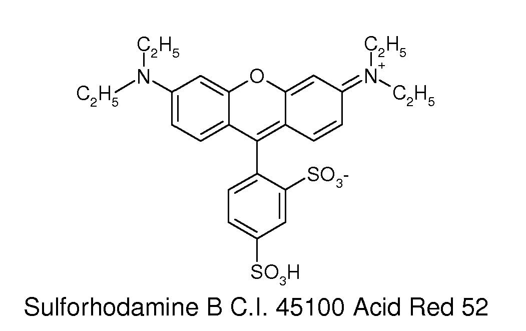
- Les derives de thioxanthènes tels que 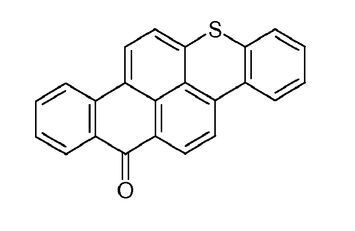 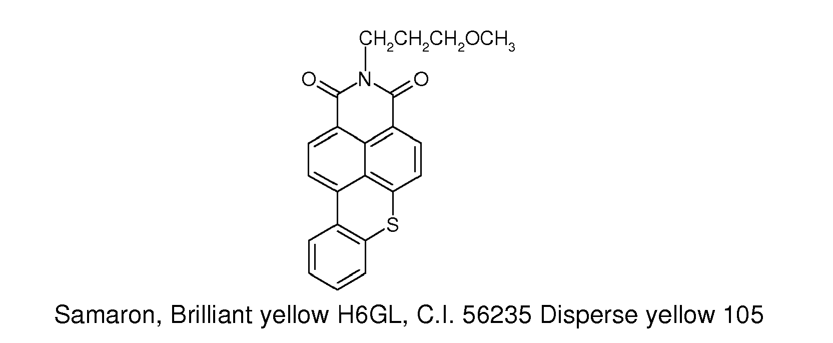
- Les dérivés de naphtolactames tels que : 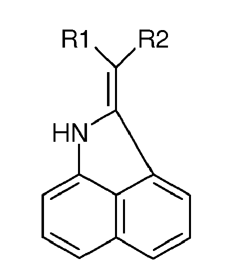 avec R 1 et R2 définis comme précédemment

On peut citer par exemple le composé suivant 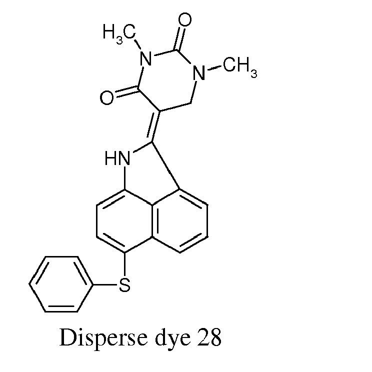
- Les dérivés AZALACTONES : 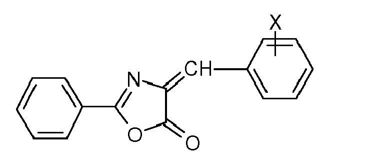 X ayant la même définition que R1 décrit précédemment.

On peut citer par exemple le composé suivant 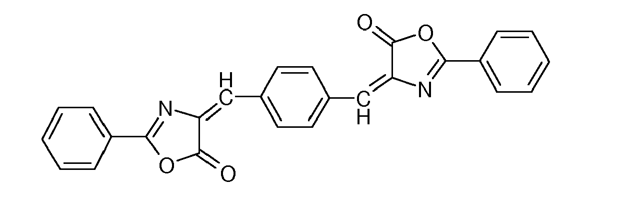
- Les dérivés méthiniques tels que 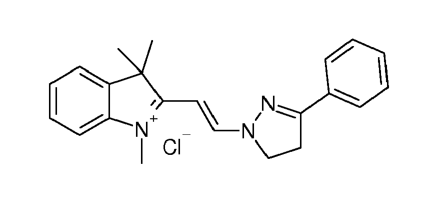 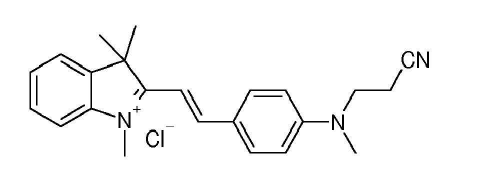
- Les dérivés oxazines et thiazines tels que 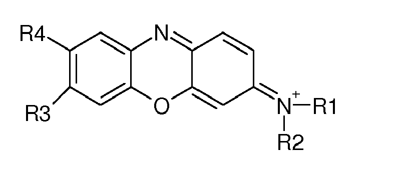
dans lesquelles R1, R2, R3 et R4 sont tels que définis ci-dessus. A titre d'exemple, on peut citer 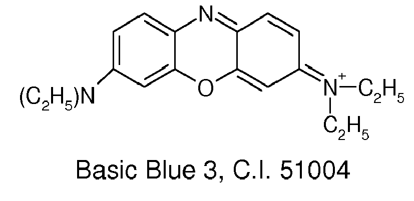
- Les dérives de 1,4-DISTYLBENZENES de formule : 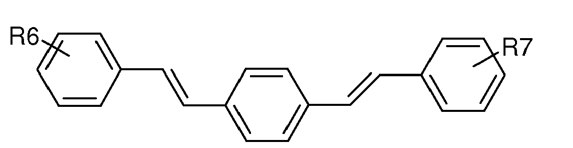
dans laquelle R6 et R7 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6 ; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, aryle éventuellement substitué, carboxyle, sulfo, un atome d'halogène,ce radical alkyle pouvant être interrompu par un hétéroatome, tel que N, S ou O.
- Les dérivés 4,4'-DISTYRYLBIPHENYLES de formule : 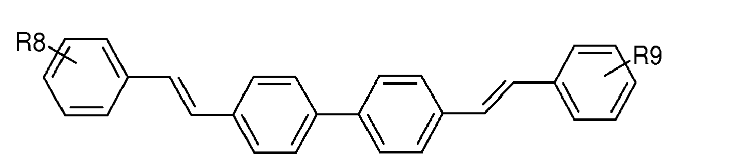
dans laquelle R8 et R9 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6 ; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène.
- Les dérivés TRIAZINYLAMINOSTILBENES de formule : 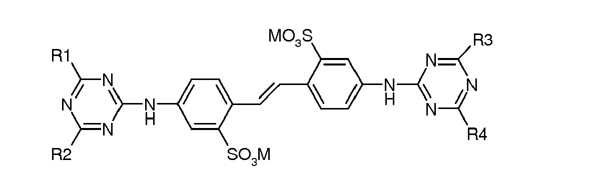
dans laquelle R1, R2, R3 et R4 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6 ; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène ; et M est un cation monovalent ou divalent issu de la famille des alcalins ou des alcalino-terreux, comme par exemple les ions sodium, potassium et calcium.
- Les dérivés STILBAZOLIUM de formule: 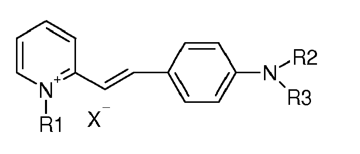
dans laquelle R1, R2 et R3 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6 ; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène ; deux des substituants R2 , R3 peuvent former avec les atomes de carbone auxquels ils sont rattachés un noyau aromatique en C6-C30 ou hétérocyclique comportant au total de 5 à 30 chaînons et de 1 à 5 hétéroatomes ; ce cycle étant condensés ou non, ce cycle et l'éventuel cycle condensé étant substitués ou non par au moins un groupement choisi parmi les groupements alkyle en C1-C4, alcoxy(C1-C4)alkyl(C1-C4), amino, dialkyl(C1-C4)amino, halogène, phényle, carboxy, trialkyl(C1-C4)ammonioalkyl(C1-C4), et
X- est un anion organique ou minéral. On peut citer par exemple pour X-les ions Chlorure, Bromure, Iodure, méthosulfate, éthosulfate, mésylate, tosylate, acétate, les sels d'acide organique simple tel que les lactate, les oléate, les benzoate, les perchlorate, les triflate.
- Les Dimères de Stilbazolium de formules : 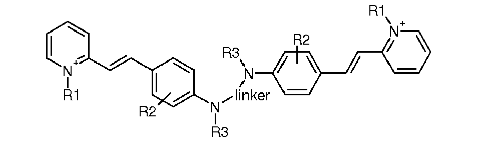 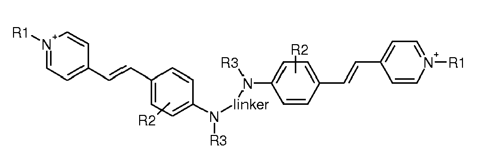
dans lesquelles R1, R2, R3 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6 ; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène.
- Les Trimères et tétramères de Stilbazolium suivants 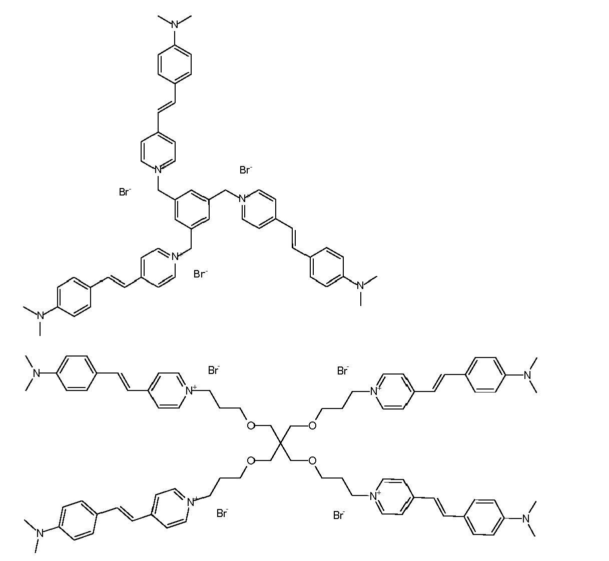
- Les dérivés Bis(BENZOXAZOLE) de formule : 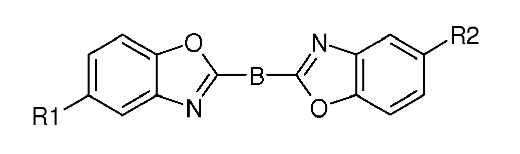
dans laquelle R1, R2 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6 ; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène ; et B est choisi parmi 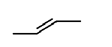 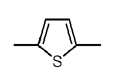 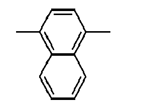 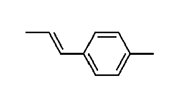
- Les Bis(BENZIMIDAZOLES) cationiques ou non
- Les 1,3-Diphenyl-2-PYRAZOLINES anioniques ou non, notamment 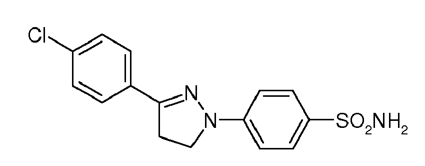
- Les DICETOPYRROLOPYRROLE de formule : 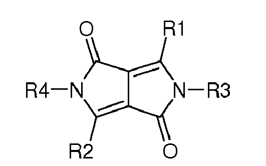
dans laquelle R1, R2, R3 et R4 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6 ; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle, amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène.
On peut citer par exemple le composé suivant 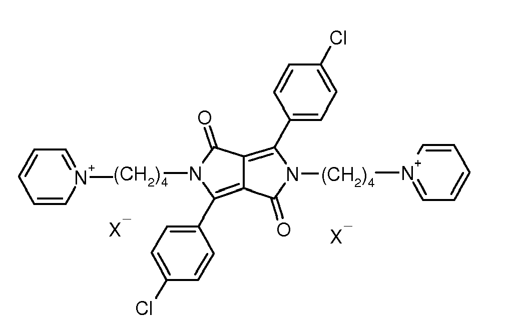 dans lequel X⁻ est un anion défini comme précédemment.
- Les dérivés de DICYANO PYRAZINES de formules : 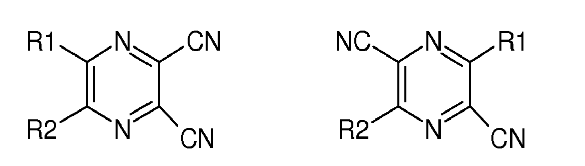
dans lesquelles R1, R2 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6 ; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène ; deux des substituants R1 et R2, peuvent former avec les atomes de carbone auxquels ils sont rattachés un cycle aromatique ou non, en C6-C30 ou hétérocyclique comportant au total de 5 à 30 chaînons et de 1 à 5 hétéroatomes ; ces cycles étant condensés ou non,insérant ou non un groupement carbonyle, et étant substitués ou non par un ou plusieurs groupements choisis parmi les groupements alkyle en C1-C4, alcoxy(C1-C4)alkyl(C1-C4), amino, dialkyl(C1-C4)amino, halogène, phényle, carboxy, trialkyl(C1-C4)ammonioalkyl(C1-C4) ; deux des substituants R2 , R3 peuvent former avec les atomes de carbone auxquels ils sont rattachés un noyau aromatique en C6-C30 ou hétérocyclique comportant au total de 5 à 30 chaînons et de 1 à 5 hétéroatomes ; ce cycle étant condensés ou non,ce cycle et l'éventuel cycle condensé étant substitués ou non par au moins un groupement choisi parmi les groupements alkyle en C1-C4, alcoxy(C1-C4)alkyl(C1-C4), amino, dialkyl(C1-C4)amino, halogène, phényle, carboxy, trialkyl(C1-C4)ammonioalkyl(C1-C4).

On peut citer les composés dans la publication suivante : « Selective topochemicalphotoreaction of crystallized 2,3-(-phenyletheny)- 4,5-dicyanopyrazine" de Kim, Jae Hong; Matsuoka Masaru, Chem. Lett. (1999), (2), 143-144

On peut notamment citer

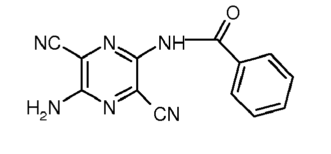

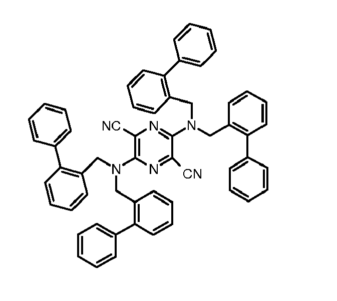

La quantité de composés fluorescents solubles utiles dans la présente invention peuvent varier dans des gammes très variées. A titre d'exemple la quantité de composés fluorescents peut être comprise entre 0,01 et 40 %, préférentiellement entre 0,05 et 20 % et plus particulièrement encore de 0,1 à 10% environ en poids du poids total de la composition.

Le ou les monomères électrophiles présents dans la composition de l'invention peuvent être choisis parmi
- les dérivés benzylidene malononitrile (A), le 2-(4-chloro-benzylidene)-malononitrile (A1) le 2-cyano-3-phényl acrylate d'éthyle (B) le, 2-cyano-3-(4-chloro-phényl) acrylate d'éthyle (B1) décrits dans Sayyah, J. Polymer Research, 2000, p97

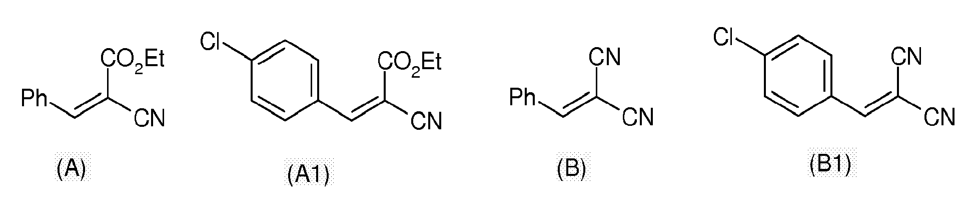

Les dérivés de méthylidenemalonates comme :
■ Le 2-méthylene-malonate de diéthyle (C) par Hopff, Makromoleculare Chemie, 1961, p95, De Keyser, J. Pharm. Sci, 1991, p67 et Klemarczyk, Polymer, 1998, p173 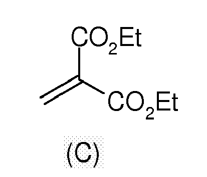
■ le 2-éthoxycarbonylméthyleneoxycarbonyl acrylate d'éthyle (D) par Breton, Biomaterials, 1998, p271 et Couvreur, Pharmaceutical Research, 1994, p1270.

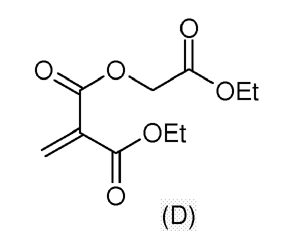

Les dérivés itaconate et itaconimide comme :
■ l'itaconate de diméthyle (E) par Bachrach, European Polymer Journal, 1976, p563 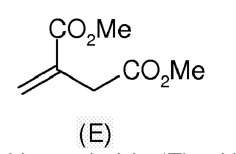
■ N-butyl itaconimide (F) , N-(4-tolyl) itaconimide (G) , N-(2-ethylphenyl) itaconimide (H) , N-(2,6-diethylphenyl) itaconimide (I) par Wanatabe, J.Polymer Science : Part A :Polymer chemistry, 1994, p2073

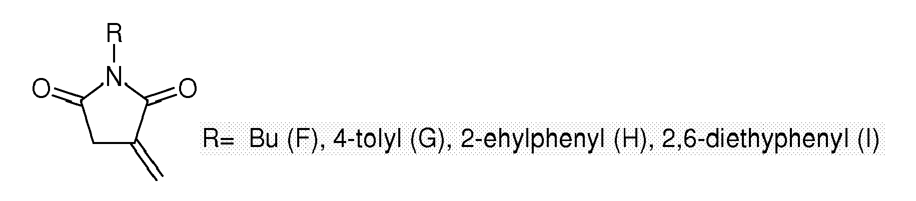

Les dérivés α-(methylsulfonyl)acrylates de méthyle (K) , α-(methylsulfonyl)acrylates de d'éthyle (L) , α-(tert-butylsulfonyl)acrylates de méthyle (M) , α-(methylsulfonyl)acrylates de tert-butyle (N) α-( tert-butylsulfonyl)acrylates de tert-butyle (O) par Gipstein, J.Org.Chem, 1980, p1486 et
les dérivés 1,1-bis-(methylsulfonyl)ethylene (P), 1-acetyl-1-methyl sulfonyl ethylene (Q) , α-(methylsulfonyl) vinyl sulfonate de methyle (R) , α-methylsulfonylacrylonitrile (S) par Shearer, US patent US2748050.

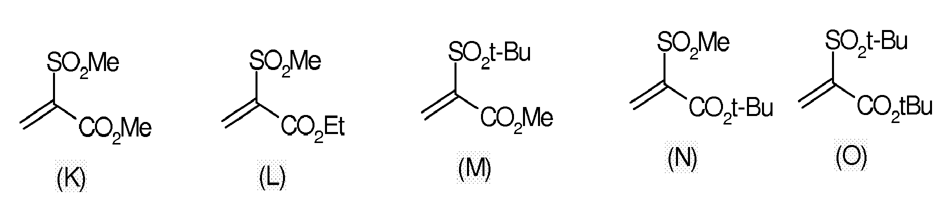

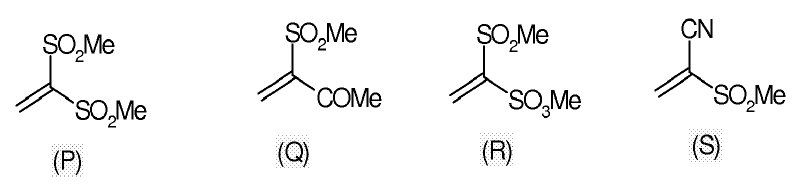

Les dérivés méthyl vinyl sulfone (T) et phényl vinyl sulfone (U) par Boor , J. Polymer Science, 1971, p249

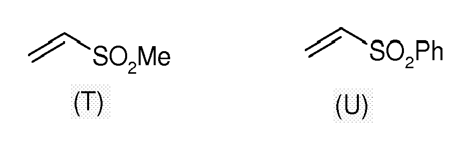

Le dérivé phényl vinyl sulfoxide (V) par Kanga, Polymer preprints (ACS, Divison of Polymer Chemistry), 1987, p322

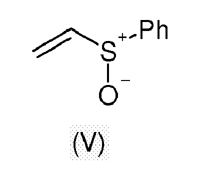

Le dérivé 3-methyl-N-(phenylsulfonyl)-1-aza-1,3-butadiene (W) par Bonner, Polymer Bulletin, 1992, p517

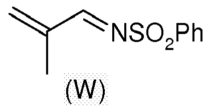

Les dérivés acrylates et acrylamides comme :
■ N-propyl-N-(3-triisopropoxysilylpropyl)acrylamide (X) et N-propyl-N-(3-triethoxysilylpropyl)acrylamide (Y) par Kobayashi, Journal of Polymer Science, Part A: Polymer Chemistry, 2005, p2754. 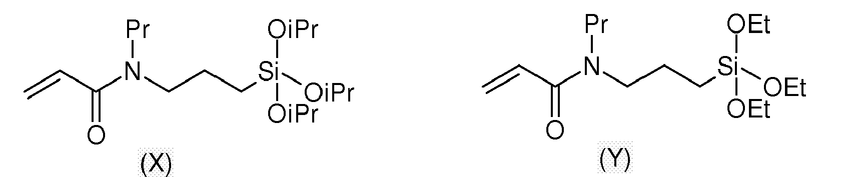
■ 2-hydroxyethyl acrylate (Z) et 2-hydroxyethyl méthacrylate (AA) par Rozenberg, International Journal of Plastics Technology, 2003, p17 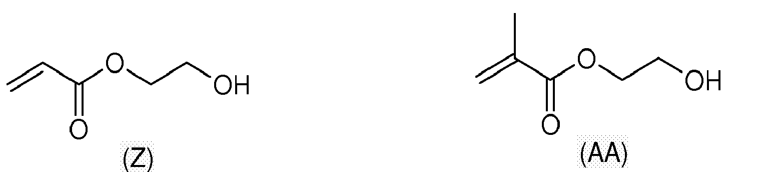
■ N-butyl acrylate (AB) par Schmitt, Macromolecules, 2001, p2115
■ Tert-butyl acrylate (AC) par lshizone, Macromolecules, 1999, p955.

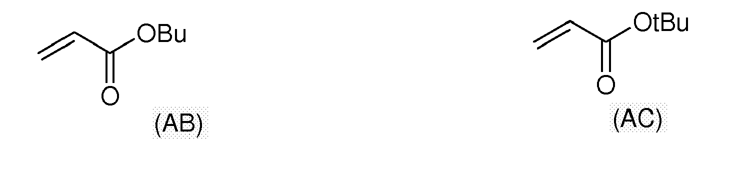

Le monomère électro-attracteur utile dans la présente invention peut être cyclique ou linéaire. Lorsqu'il est cyclique, le groupe éléctro-attracteur est de préférence exocyclique, c'est-à-dire qu'il ne fait pas partie intégrante de la structure cyclique du monomère.

Selon un mode de réalisation particulier, ces monomères présentent au moins deux groupes électro-attracteurs.

A titre d'exemple de monomères présentant au moins deux groupes électro-attracteurs, on peut citer les monomères de formule (A) : 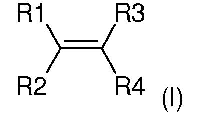 dans laquelle :
- R1 et R2 désignent chacun, indépendamment l'un de l'autre, un groupe peu ou non électro-attracteur (peu ou non inductif-attracteur) tel que :
   - un atome d'hydrogène,
   - un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR, -OH, et les atomes d'halogène,
   - un résidu polyorganosiloxane modifié ou non,
   - un groupement polyoxyalkylène,
- R3 et R4 désignent chacun, indépendamment l'un de l'autre, un groupe électro-attracteur (ou inductif-attacteur) choisi de préférence parmi les groupements -N(R)3+, -S(R)2+, -SH2+, -NH3+, -NO2, - SO2R, -C≡N, -COOH, -COOR, -COSR, -CONH2, CONHR, -F, -Cl, - Br, -I, -OR, -COR, -SH, -SR, -OH, les groupes alcényle linéaires ou ramifiés, les groupes alcynyle linéaires ou ramifiés, les groupements mono- ou polyfluoroalkyle en C1-C4, les groupements aryle tels que phényle, ou les groupements aryloxy tels que phénoxyloxy,
- R désigne un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère pouvant être obtenu par polymérisation radicalaire, par polycondensation ou par ouverture de cycle, R' désignant un groupe alkyle en C₁-C₁₀.

Par groupement électro-attracteur ou inductif-attracteur (-I), on entend tout groupement plus électronégatif que le carbone. On pourra se reporter à l'ouvrage PR Wells Prog. Phys. Org. Chem., Vol 6,111 (1968).

Par groupement peu ou non électro-attracteur, on entend tout groupement dont l'électronégativité est inférieure ou égale à celle du carbone.

Les groupements alcényle ou alcynyle ont de préférence 2 à 20 atomes de carbone, mieux encore de 2 à 10 atomes de carbone.

Comme groupe hydrocarboné saturé ou non, linéaire, ramifié ou cyclique, comportant de préférence de 1 à 20 atomes de carbone, on peut notamment citer les groupes alkyle, alcényle ou alcynyle linéaires ou ramifiés, tels que méthyle, éthyle, n-butyle, tert-butyle, iso-butyle, pentyle, hexyle, octyle, butényle ou butynyle ; les groupes cycloalkyle ou aromatiques.

Comme groupe hydrocarboné substitué, on peut citer par exemple les groupes hydroxyalkyle ou polyhalogénoalkyle.

A titre d'exemples de polyorganosiloxane non modifié, on peut notamment citer les polyalkylsiloxanes tels que les polydiméthylsiloxanes, les polyarylsiloxanes tels que les polyphénylsiloxanes, les polyarylalkylsiloxanes tels que les polyméthylphénylsiloxanes.

Parmi les polyorganosiloxanes modifiés, on peut notamment citer les polydiméthylsiloxanes à groupements polyoxyalkylène et/ou siloxy et/ou silanol et/ou amine et/ou imine et/ou fluoroalkyle.

Parmi les groupements polyoxyalkylène, on peut notamment citer les groupements polyoxyéthylène et les groupements polyoxypropylène ayant de préférence 1 à 200 motifs oxyalkylénés.

Parmi les groupements mono- ou polyfluoroalkyle, on peut notamment citer des groupements tels que -(CH2)n-(CF2)m-CF3 ou - (CH2)n-(CF2)m-CHF2 avec n=1 à 20 et m= 1 à 20.

Les substituants R1 à R4 peuvent éventuellement être substitués par un groupement ayant une activité cosmétique. Les activités cosmétiques particulièrement utilisées sont obtenues à partir de groupements à fonctions colorantes, antioxydantes, filtres UV et conditionnantes.

A titre d'exemples de groupement à fonction colorante, on peut notamment citer les groupements azoïques, quinoniques, méthiniques, cyanométhiniques et triarylméthane.

A titre d'exemples de groupement à fonction antioxydante, on peut notamment citer les groupements de type butylhydroxyanisole (BHA), butylhydroxytoluène (BHT) ou vitamine E.

A titre d'exemples de groupement à fonction filtre UV, on peut notamment citer les groupements de types benzophénones, cinnamates, benzoates, benzylidène-camphres et dibenzoylméthanes.

A titre d'exemples de groupement à fonction conditionnante, on peut notamment citer les groupements cationiques et de type esters gras.

Parmi les monomères précédemment cités, sont préférés les monomères de la famille des cyanoacrylates et leurs dérivés de formule (II) : 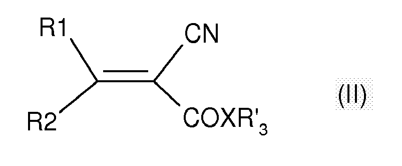 X désignant NH, S ou O,
R1 et R2 ayant les mêmes significations que précédemment, de préférence R1 et R2 représentant un atome d'hydrogène,
R'3 représentant un atome d'hydrogène ou R tel que défini à la formule (I).

De préférence, X désigne O.

A titre de composés de formule (II), on peut citer les monomères :
a) appartenant à la famille des 2-cyanoacrylates de polyfluoroalkyle tels que :
   l'ester 2,2,3,3-tétrafluoropropylique de l'acide 2-cyano-2-propénoïque de formule : 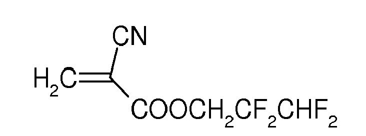
   ou encore l'ester 2,2,2-trifluoroéthylique de l'acide 2-cyano-2-propénoïque de formule : 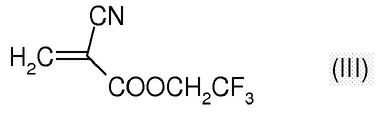
b) les 2-cyanoacrylates d'alkyle ou d'alcoxyalkyle 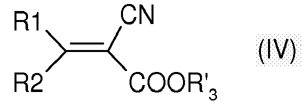
dans laquelle R'3 représente un radical alkyle en C1-C10 ou alcoxy(C1-C4) alkyle(C1-C10) ou alcényle en C2-C10.

On peut citer plus particulièrement le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle, le 2-cyanoacrylate d'allyle, le 2-cyanoacrylate de méthoxypropyle.

Dans le cadre de l'invention, on préfère utiliser les monomères b). Selon un mode de réalisation préféré, le ou les monomères cyanoacrylates sont choisis parmi les cyanoacrylates d'alkyle en C6-C10.

Les monomères particulièrement préférés sont les cyanoacrylates d'octyle de formule V et leurs mélanges : 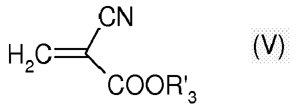 dans laquelle :
- R'₃ =: -(CH₂)₇-CH₃,
-CH(CH₃)-(CH₂)₅-CH₃,
-CH₂-CH(C₂H₅)-(CH₂)₃-CH₃,
-(CH₂)₅-CH(CH₃)-CH₃,
-(CH₂)₄-CH(C₂H₅)-CH₃

Les monomères utilisés conformément à l'invention peuvent être fixés de façon covalente sur des supports tels que des polymères, des oligomères ou des dendrimères. Le polymère ou l'oligomère peut être linéaire, ramifié, en peigne ou bloc. La répartition des monomères de l'invention sur la structure polymérique, oligomérique ou dendritique peut être statistique, en position terminale ou sous forme de blocs.

Dans la composition de l'invention, la quantité de monomères électrophiles tels que les cyanoacrylates est comprise entre 0,1 et 80 % en poids du poids total de la composition, de préférence entre 1 et 50 %.

Dans le cadre de l'invention, les monomères électrophiles sont des monomères capables de polymériser par voie anionique en présence d'un agent nucléophile. Par polymérisation anionique, on entend le mécanisme défini dans l'ouvrage "Advanced Organic Chemistry", Third Edition de Jerry March, pages 151 à 161.

Les agents nucléophiles susceptibles d'initier la polymérisation anionique sont des systèmes connus en eux-mêmes, capables de générer un carbanion au contact d'un agent nucléophile, tels que les ions hydroxyles contenus dans l'eau. On entend par "carbanion ", les espèces chimiques définies dans " Advanced Organic Chemistry, Third Edition ", de Jerry March, page 141.

Les agents nucléophiles peuvent être appliqués indépendamment de la composition de l'invention. Ils peuvent aussi être ajoutés à la composition de l'invention au moment de l'emploi.

L'agent nucléophile est un composé moléculaire, un oligomère, un dendrimère ou un polymère possédant des fonctions nucléophiles. De façon non limitative, on peut citer comme fonctions nucléophiles les fonctions : R₂N⁻, NH₂⁻, Ph₃C⁻, R₃C⁻, PHNH⁻, pyridine, ArS⁻, R-C^{≡}C⁻, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻, H₂O, Ph représentant un groupe phényle ; Ar représentant un groupe aryle et R représentant un groupe alkyle en C1-C10.

Les monomères électrophiles peuvent être synthétisés selon les méthodes connues décrites dans la technique. En particulier, les monomères cyanoacrylates peuvent être synthétisés selon l'enseignement du US 3 527 224, US 3 591 767, US 3 667 472, US 3 995 641, US 4 035 334 et US 4 650 826.

Par solvant organique, on entend une substance organique capable de dissoudre une autre substance sans la modifier chimiquement.

Le ou les solvants organiques liquides présents dans la composition de l'invention sont choisis parmi les composés liquides à la température de 25°C et sous 105 Pa (760mm de Hg).

Dans le cadre de l'invention, les monomères électrophiles et le solvant organiques sont des composés distincts.

Le solvant organique est par exemple choisi parmi :

les alcools aromatiques tels que l'alcool benzylique ; les alcools gras liquides , notamment en C₁₀-C₃₀; les polyols modifiés ou non tels que le glycérol, le glycol, le propylène glycol, le dipropylène glycol, le butylène glycol, le butyle diglycol ; les silicones volatiles telles que la cylopentasiloxane, la cyclohexasiloxane, les polydiméthylsiloxanes modifiées ou non par des fonctions alkyle et/ou amine et/ou imine et/ou fluoroalkyl et/ou carboxylique et/ou betaïne et/ou ammonium quaternaire, les polydiméthylsiloxanes modifiées liquides, les huiles minérales, organiques ou végétales, les alcanes et plus particulièrement les alcanes de C₅ à C_{10;} les acides gras liquides, les esters gras liquides et plus particulièrement les benzoates ou les salicylates d'alcool gras liquides.

Le solvant organique est de préférence choisi parmi les huiles organiques ; les silicones telles que les silicones volatiles, les gommes ou huiles de silicones aminés ou non et leurs mélanges ; les huiles minérales ; les huiles végétales telles que les huiles d'olive, de ricin, de colza, de coprah, de germe de blé, d'amande douce, d'avocat, de macadamia, d'abricot, de carthame, de noix de bancoulier, de camélina, de tamanu, de citron ou encore des composés organiques tels que des alcanes en C₅-C₁₀, l'acétone, la méthyléthylcétone, les esters d'acides en C₁-C₂₀ liquides et d'alcools en C₁-C₈ tels que l'acétate de méthyle, l'acétate de butyle, l'acétate d'éthyle et le myristate d'isopropyle, le diméthoxyéthane, le diéthoxyéthane, les alcools gras liquides en C₁₀-C₃₀ tels que l'alcool oléique, les esters d'alcools gras en C₁₀-C₃₀ liquides tels que les benzoates d'alcool gras en C₁₀-C₃₀ et leurs mélanges ; l'huile de polybutène, l'isononanoate d'isononyle, le malate d'isostéaryle, le tétra-isostéarate de pentaérythrityle, le trimélate de tridécyle, le mélange cyclopentasiloxane (14,7% en poids)/polydiméthylsiloxane dihydroxylé en positions α et ω (85,3% en poids), ou leurs mélanges.

Selon un mode de réalisation préféré, le solvant organique liquide est constitué par une silicone ou un mélange de silicones tels que les polydiméthylsiloxanes liquides et les polydiméthylsiloxanes modifiées liquides, leur viscosité à 25°c est comprise entre 0.1 cst et 1 000 000 cst et plus préférentiellement entre 1 cst et 30 000cst.

On citera de préférence les huiles et mélanges d'huiles suivantes :
- le mélange de polydiméthylsiloxane alpha-omega-dihydroxylé/cyclopentadiméthylsiloxane (14,7/85,3) commercialisé par Dow Corning sous le nom de DC 1501 Fluid
- le mélange de polydiméthylsiloxane alpha-omega-dihydroxylé/ polydiméthylsiloxane commercialisé par Dow Corning sous le nom de DC 1503 Fluid
- le mélange de diméthicone /cyclopentadiméthylsiloxane commercialisé par Dow Corning sous le nom de DC 1411 Fluid ou celui commercialisé par Bayer sous le nom SF1214 ;
- la cyclopentadiméthylsiloxane commercialisée par Dow Corning sous le nom de DC245 Fluid ; et les mélanges respectifs de ces huiles.

Le milieu des compositions de l'invention peut contenir, outre le ou les solvants organiques liquides, de l'eau.

De préférence c'est un milieu anhydre c'est-à-dire contenant moins de 1% en poids d'eau par rapport au poids total de la composition.

Le ou les solvants organiques de la composition représentent généralement de 0,01 à 99 %, de préférence de 50 à 99 % en poids par rapport au poids total de la composition.

La composition de l'invention peut se présenter sous forme d'une émulsion et/ou être encapsulé, les monomères électrophiles étant maintenus dans un milieu anhydre jusqu'au moment de l'utilisation. Lorsque la composition est une émulsion, cette émulsion est par exemple constituée par une phase dispersée ou continue qui peut être constituée par de l'eau, des alcools aliphatiques en C1-C4 ou leurs mélanges et une phase organique anhydre comprenant le monomère. Dans le cas des capsules ou microcapsules, la capsule peut contenir le monomères dans un milieu anhydre et être dispersées dans un milieu anhydre tel que défini précédemment, de l'eau, des alcools aliphatiques en C1-C4 ou leurs mélanges.

On peut introduire dans les compositions des inhibiteurs de polymérisation, et plus particulièrement des inhibiteurs de polymérisation anioniques et/ou radicalaires, ceci afin d'accroître la stabilité de la composition dans le temps. De façon non limitative, on peut citer les inhibiteurs de polymérisation suivants : le dioxyde de soufre, l'oxyde nitrique, le trifluorure de bore, l'hydroquinone et ses dérivés tels que l'hydroquinone monéthyléther, la TBHQ, la benzoquinone et ses dérivés tels que la duroquinone, le catéchol et ses dérivés tels que le t-butyl catéchol et le méthoxycatéchol, l'anisole et ses dérivés tels que le méthoxyanisole ou l'hydroxyanisole, le pyrogallol et ses dérivés, le p-méthoxyphénol, l'hydroxybutyl toluène, les alkyl sulfates, les alkyl sulfites, les alkyl sulfones, les alkyl sulfoxydes, les alkyl sulfures, les mercaptans, le 3-sulfonène et leurs mélanges. Les groupements alkyle désignent de préférence des groupement ayant 1 à 6 atomes de carbone.

On peut aussi utiliser à titre d'inhibiteur les acides minéraux ou organiques.

Ainsi la composition cosmétique selon l'invention peut également comprendre au moins un acide minéral ou organique, ce dernier ayant un ou plusieurs groupements carboxyliques ou sulfoniques, présentant un pKa compris entre 0 et 6 tels que l'acide phosphorique, l'acide chlorhydrique, l'acide nitrique, l'acide benzène- ou toluène-sulfonique, l'acide sulfurique, l'acide carbonique, l'acide fluorhydrique, l'acide acétique, l'acide formique, l'acide propionique, l'acide benzoïque, les acides mono-, di- ou trichloroacétiques, l'acide salicylique et l'acide trifluoroacétique, l'acide octanoïque, l'acide heptanoïque et l'acide hexanoïque.

De préférence, l'acide acétique est utilisé.

La concentration en inhibiteur dans la composition cosmétique de l'invention peut être comprise entre 10 ppm et 30% en poids et plus préférentiellement entre 10 ppm et 15% en poids par rapport au poids total de la composition.

La composition de l'invention peut aussi contenir un ou plusieurs pigments. Elle peut aussi contenir des poudres ou particules métalliques telles que des poudres ou particules d'aluminium, de zinc, de cuivre, etc.

La composition peut aussi contenir les actifs cosmétiques habituellement utilisés. On peut citer à titre non limitatif les charges, les agents réducteurs, les agents oxydants, les corps gras, les silicones, les agents épaississants, les adoucissants, les agents anti-mousse, les agents hydratants, les agents émollients, les agents alcalinisants, les élastomères, les plastifiants, les filtres solaires, les colorants directs non fluorescents, le colorants d'oxydation, les argiles, les minéraux colloïdaux, les parfums, les peptisants, les conservateurs, les tensioactifs anioniques cationiques, amphotères, zwitterioniques ou non-ioniques, les polymères fixants ou non, les polymères conditionneurs, les élastomères, les protéines, les vitamines.

Ces compositions peuvent se présenter sous des formes diverses, telles que des lotions, des sprays, des mousses et être appliquées sous forme de shampooing ou d'après-shampooing.

Dans le cas des sprays, la composition de l'invention peut contenir un propulseur. Le propulseur est constitué par les gaz comprimés ou liquéfiés usuellement employés pour la préparation de compositions aérosols. On emploiera de manière préférentielle l'air, le gaz carbonique, l'azote comprimé ou encore un gaz soluble tel que le diméthyléther, les hydrocarbures halogénés (fluorés en particuliers) ou non et leurs mélanges.

Selon le procédé de l'invention, la composition de l'invention est appliquée sur les fibres kératiniques, en particulier les cheveux, en présence d'un agent nucléophile.

Selon un mode de réalisation particulier du procédé de l'invention, l'agent nucléophile capable d'initier la polymérisation du monomère cyanoacrylate peut être appliqué au préalable sur les fibres kératiniques. L'agent nucléophile peut être utilisé pur, en solution, sous forme d'une émulsion ou être encapsulé. II peut aussi être ajouté à la composition anhydre au moment de l'emploi juste avant l'application sur les fibres kératiniques.

De préférence, cet agent nucléophile est l'eau. Cette eau peut être apportée par exemple par humidification préalable des fibres kératiniques. Elle peut aussi être ajoutée directement dans la composition avant application.

Selon un mode de réalisation particulier, il est possible de moduler la cinétique de polymérisation en humidifiant préalablement la fibre à l'aide d'une solution aqueuse dont le pH a été ajusté à l'aide d'une base, d'un acide ou d'un mélange acide/base. L'acide et/ou la base peuvent être inorganique ou organique.

Selon une variante, le procédé de l'invention peut être mise en oeuvre en plusieurs étapes : une première étape qui consiste à appliquer une composition contenant le ou les composé fluorescents sur les fibres et une seconde étape qui consiste à appliquer une composition contenant le monomère électrophile, l'agent nucléophile étant présent dans la composition contenant le composé fluorescent ou dans une composition séparée, le solvant organique liquide étant dans l'une et/ou l'autre des compositions.

Selon cette variante, la composition contenant le ou les composés fluorescents est de préférence une solution aqueuse de composés fluorescents ce qui permet une humidification de la fibre et l'initiation de la polymérisation lorsque le monomère électrophile est appliqué.

Selon le procédé de l'invention, un mode de réalisation préféré consiste à appliquer soit le monomère électrophile et les composés fluorescents à partir d'une même composition soit d'appliquer dans un premier temps le composé fluorescent, puis le monomère électrophile , le solvant organique étant présent dans l'une et/ou l'autre des compositions.

Le procédé de l'invention peut comprendre des étapes additionnels intermédiaires ou finales telles que l'application d'un produit cosmétique, une étape de rinçage, une étape de séchage. Le séchage peut être effectué au casque, au sèche cheveux et/ou au fer à lisser. En particulier, l'application des compositions conformes à l'invention peut être suivie d'un rinçage.

Il est aussi possible de réaliser des applications multiples de la composition de l'invention afin d'obtenir une superposition de couches pour atteindre des propriétés spécifiques du dépôt en termes de nature chimique, résistance mécanique, épaisseur, aspect, toucher.

Afin d'améliorer entre autre l'adhésion du poly(cyanoacrylate) formé in situ, la fibre peut être prétraitée avec tous types de polymères.

Pour moduler la cinétique de polymérisation anionique, on peut également augmenter la nucléophilie de la fibre par transformation chimique des fibres kératiniques. A titre d'exemple, on peut citer la réduction des ponts di-sulfure composant en partie la kératine en thiols avant application de la composition de l'invention. De façon non exhaustive, on peut citer comme réducteurs des ponts di-sulfure composant en partie la kératine, les composés suivants: thiosulfate de sodium anhydre, métabisulfite de sodium en poudre, thiourée, sulfite d'ammonium, acide thioglycolique, acide thiolactique, thiolactate d'ammonium, mono-thioglycolate de glycérol, thioglycolate d'ammonium, thioglycérol, acide 2,5-dihydroxybenzoique, di-thioglycolate de diammonium, thioglycolate de strontium, thioglycolate de calcium, formo-sulfoxylate de zinc, thioglycolate d'isooctyle, dl-cystéine, thioglycolate de monoéthanolamine.

L'application de la composition de l'invention peut aussi être précédée d'un traitement capillaire comme une coloration directe ou d'oxydation.

Selon la présente invention, les monomères sont de préférence choisis parmi les monomères capables de polymériser sur les fibres kératiniques dans des conditions cosmétiquement acceptables. En particulier, la polymérisation du monomère s'effectue de préférence à une température inférieure ou égale à 80°C ce qui n'empêche pas de terminer l'application par un séchage au casque, un brushing ou passage au fer plat ou à friser.

Selon un mode de réalisation particulier du procédé de la présente invention, la composition de l'invention est appliquée sur des cheveux foncés. En particulier, la composition cosmétique de l'invention permet de teindre avec un effet éclaircissant des cheveux pigmentés ou colorés artificiellement et dont la hauteur de ton est inférieure ou égale à 6 et de préférence inférieure ou égale à 4. La hauteur de ton est une caractéristique bien connue de la technique de la coloration. Elle est par exemple décrite dans l'ouvrage « Science des traitements capillaires », Charles ZVIAK, édition Masson 1988, page 278.

L'invention a aussi pour objet un kit de coloration comprenant une première composition qui contient au moins un composé fluorescent soluble et une seconde composition qui contient le ou les monomères électrophiles et éventuellement une troisième composition qui contient l'agent nucléophile, le solvant organique étant présent dans l'un et/ou l'autre des trois compartiments.

Selon une première variante, le kit comprend une première composition qui comprend le ou les composés fluorescents solubles et une deuxième composition qui comprend le ou les monomères électrophiles et le solvant organique liquide. Selon un mode de réalisation de cette variante, la composition contenant le ou les composés fluorescents est une composition aqueuse et la composition contenant le ou les monomères est une composition anhydre.

### EXEMPLES

### Exemple 1 : Famille des pyrazines

La composition suivante a été réalisée :

| **Ingrédient** | **Quantité** |
|---|---|
| poly diméthyl siloxane alpha-omega dihydroxylé / cyclopentadiméthylsiloxane (14,7/85,3) commercialisé par Dow Corning sous le nom de DC 1501 Fluid | 40 g |
| cyclopentadiméthylsiloxane commercialisé par Dow Corning sous le nom de DC245 Fluid | 40 g |
| Composé 1 : 5-chloro-8-benzylminopyrazino(2,3-d)-pyridazine* | 10g |
| Méthylheptylcyanoacrylate fabriqué par la société Chemence | 10 g |
| Acide acétique | 0,25g |

| | |
|---|---|
| * cité dans les publications suivantes : Natu R.Patel, Journal of Heterocyclic Chemistry, Vol3, N°4, 1966, pp512-517 ; Kazuko Shirai, Journal of the Society of Dyes and Colorists, Vol114, n °12, Dec 1998, pp368-374. | |

0,5 g de cette composition est appliqué sur une mèche de cheveux châtains naturels de hauteur de ton 4, propres et humides de 1 g. Après 15 minutes de pose à température ambiante, la mèche est séchée au sèche-cheveux pendant 2 minutes.
La mèche est colorée en jaune-orange intense et la coloration obtenue est résistante aux shampooings. La coloration est très fluorescente à la lumière du jour. La mèche obtenue présente un toucher doux.

### Exemple 2 : Famille des pyrazines

La composition suivante est réalisée :

| **Ingrédient** | **Quantité** |
|---|---|
| poly diméthyl siloxane alpha-omega dihydroxylé / cyclopentadiméthylsiloxane (14,7 / 85,3) commercialisé par Dow Corning sous le nom de DC 1501 Fluid | 40 g |
| cyclopentadiméthylsiloxane commercialisé par Dow Corning sous le nom de DC245 Fluid | 40 g |
| Composé 2 : 2,3-dicyano-5-hydroxy-6-(2-julolidinyl-éthényl)-pyrazine* | 10 g |
| Méthylheptylcyanoacrylate fabriqué par la société Chemence | 10 g |

| | |
|---|---|
| * cité dans les publications suivantes : Natu R.Patel, Journal of Heterocyclic Chemistry, Vol3, N°4, 1966, pp512-517 ; Kazuko Shirai, Journal of the Society of Dyes and Colorists, Vol114, n °12, Dec 1998, pp368-374. | |

0,5 g de cette composition est appliqué sur une mèche de cheveux châtains naturels de hauteur de ton 4, propres et humides de 1 g. Après 15 minutes de pose à température ambiante, la mèche est séchée au sèche-cheveux pendant 2 minutes.
La mèche est colorée en jaune et la coloration obtenue est résistante aux shampooings. La coloration est très fluorescente lorsque irradiée par la lumière du jour. Les cheveux ont un aspect éclairci comparés aux cheveux châtains de départ. La composition tinctoriale présente une bonne stabilité dans le temps.

### Exemple 3: Famille des coumarines

La composition suivante est réalisée :

| **Ingrédient** | **Quantité** |
|---|---|
| poly diméthyl siloxane alpha-omega dihydroxylé / cyclopentadiméthylsiloxane (14,7 / 85,3) commercialisé par Dow Corning sous le nom de DC 1501 Fluid | 40 g |
| cyclopentadiméthylsiloxane commercialisé par Dow Corning sous le nom de DC245 Fluid | 40 g |
| Composé 5 : Diéthylamino coumarine de chez CIBA | 10 g |
| Méthylheptylcyanoacrylate de Chemence | 10 g |
| Acide acétique | 0,25 g |

0,5 g de cette composition est appliqué sur une mèche de cheveux châtains naturels de hauteur de ton 4, propres et humides de 1 g. Après 15 minutes de pose à température ambiante, la mèche est séchée au sèche-cheveux pendant 2 minutes.
La mèche est colorée en rouge légèrement bleuté et la coloration obtenue est résistante aux shampooings. La coloration est très fluorescente à la lumière du jour. L'aspect visuel et le toucher de la mèche obtenue sont très satisfaisants.

### EVALUATION

La couleur des mèches a été évaluée avant et après application de la composition de l'invention dans le système L* a* b*, au moyen d'un spectrophotomètre DM3600d MINOLTA ®,. Dans ce système L*a*b*, L* représente l'intensité de la couleur, a* indique l'axe de couleur vert/rouge et b* l'axe de couleur bleu/jaune. Plus la valeur de L est faible, plus la couleur est foncée ou très intense. Plus la valeur de a* est élevée plus la nuance est rouge et plus la valeur de b* est élevée plus la nuance est bleue.

Les résultats sont reportés dans les tableaux ci dessous

| Exemples | L* | a* | b* |
|---|---|---|---|
| Mèche Hauteur de ton de 4 | 22,8 | 2,9 | 3,6 |
| EX 1 : Composé N °1 pyrazine | 39,3 | -8,2 | 14,9 |
| EX 2 :Composé N °2 pyrazine | 31,5 | 5,4 | 25,5 |
| EX 3. Diéthylamino coumarine | 23,4 | 5,2 | -5,1 |

Ces résultats montrent qu'après application des compositions 1 et 2 de l'invention, la valeur de L* représentative de l'intensité de la couleur des mèches est supérieure à celle obtenue sur les cheveux châtains de hauteur de ton 4. Ceci montre l'effet éclaircissant de la composition de l'invention sans qu'il soit nécessaire d'avoir recours à la décoloration des fibres kératiniques. Par ailleurs, on obtient ainsi des colorations rémanentes.
La mèche de cheveux colorés avec le diéthylaminocoumarine présente une couleur rouge très visible.

### Exemple 4: application en deux étapes

La composition suivante A est réalisée :

| **Ingrédient** | **Quantité** |
|---|---|
| iodure de 2-[2-(4-diméthylamino)phényl éthényl]-1 éthyl-pyridinium commercialisé sous le nom Photosensitizing Dye NK-557 par Ubichem | 5g |
| eau | 95g |

La composition suivante B est réalisée

| **Ingrédient** | **Quantité** |
|---|---|
| poly diméthyl siloxane alpha-omega dihydroxylé / cyclopentadiméthylsiloxane (14,7 / 85,3) commercialisé par Dow Corning sous le nom de DC 1501 Fluid | 45 g |
| cyclopentadiméthylsiloxane commercialisé par Dow Corning sous le nom de DC245 Fluid | 44.75 g |
| Méthylheptylcyanoacrylate de Chemence | 10 g |
| Acide acétique | 0,25 g |

0,5 g de la composition A est appliqué sur une mèche de cheveux blancs naturels, propres et humides de 1 g. Puis 0,5 g de la composition B est appliqué sur la mèche. Après 15 minutes de pose à température ambiante, la mèche est séchée au sèche-cheveux pendant 2 minutes.
La mèche est colorée en jaune et la coloration obtenue est résistante aux shampooings. La coloration est fluorescente lorsque irradiée par la lumière du jour. L'aspect visuel et le toucher de la mèche obtenue sont très satisfaisants. On obtient ainsi des colorations rémanentes.

## Revendications

1. Composition de coloration comprenant, dans un milieu de coloration approprié, au moins un monomère électrophile, au moins un composé fluorescent soluble et au moins un solvant organique liquide.

2. Composition selon la revendication 1 dans laquelle le ou les monomères électrophile sont des monomères de formule (I) : 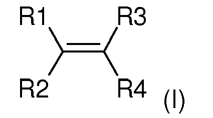 dans laquelle :
• R1 et R2 désignent chacun, indépendamment l'un de l'autre, un groupe peu ou non électro-attracteur, et
• R3 et R4 désignent chacun, indépendamment l'un de l'autre, un groupe électro-attracteur.

3. Composition selon la revendication 2 dans laquelle les monomères de formule (I) sont tels que R1 et R2, indépendamment représente un atome d'hydrogène ; un groupe hydrocarboné saturé ou non, linéaire, ramifié ou cyclique, comportant de préférence de 1 à 20 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR, -OH, et les atomes d'halogène ; un résidu polyorganosiloxane modifié ou non ; un groupement polyoxyalkylène, R désignant un groupe hydrocarboné saturé ou non, linéaire, ramifié ou cyclique, comportant de 1 à 20 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère, R'désignant un groupe alkyle en C₁-C₁₀.

4. Composition selon la revendication 2 ou 3 dans laquelle les monomères de formule (I) sont tels que R3 et R4, indépendamment sont choisis parmi les groupements -N(R)3+, -S(R)2+, -SH2+, -NH3+, -NO2, - SO2R, -C≡N, -COOH, -COOR, -COSR, -CONHR, -CONH2, -F, -Cl, -Br, -I, -OR, -COR, -SH, -SR, -OH, les groupes alcényle linéaires ou ramifiés, les groupes alcynyle linéaires ou ramifiés, les groupements mono- ou polyfluoroalkyle en C1-C4, les groupements aryle ou les groupements aryloxy , R désignant un groupe hydrocarboné saturé ou non, linéaire, ramifié ou cyclique, comportant de 1 à 20 atomes de carbone, et contenant éventuellement un ou plusieurs atomes d'azote, d'oxygène, de soufre, et éventuellement substitué par un ou plusieurs groupements choisis parmi - OR', -COOR', -COR', -SH, -SR', -OH, , les atomes d'halogène, ou un résidu de polymère, R' étant un radical alkyle en C1-C10.

5. Composition selon la revendication 1 ou 2 dans laquelle les monomères sont des monomères cyanoacrylates de formule : 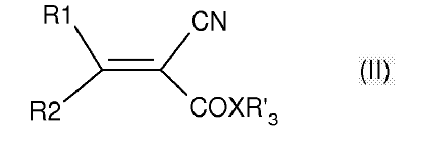 X désignant NH, S ou O,
R'₃ étant choisi parmi un atome d'hydrogène ou R,
R, R1 et R2 étant tel que défini à la revendication 3.

6. Composition selon l'une quelconque des revendications 2 à 5 dans laquelle le monomère électrophile est tel que R1 et R2 représentent un atome d'hydrogène :

7. Composition selon l'une quelconque des revendications 1 à 6 dans laquelle les monomères cyanoacrylates correspondent à la formule 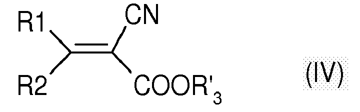 dans laquelle R'3 représente un radical alkyle en C1-C10 ou alcoxy(C1-C4)alkyle(C1-C10), alcényle en C2-C10 et R1 et R2 sont tels que définis précédemment.

8. Composition selon la revendication 6 dans laquelle R'3 est un radical alkyle comprenant de 6 à 10 atomes de carbone.

9. Composition selon la revendication 7, dans laquelle R1 et R2 représentent l'hydrogène.

10. Composition selon l'une quelconque des revendications précédentes, dans laquelle le monomère électrophile est un cyanoacrylate d'alkyle de formule 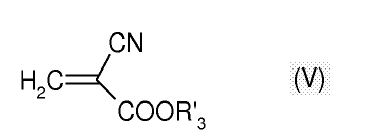 dans laquelle :
R'₃ = -(CH₂)₇-CH₃,
-CH(CH₃)-(CH₂)₅-CH₃,
-CH₂-CH(C₂H₅)-(CH₂)₃-CH₃,
-(CH₂)₅-CH(CH₃)-CH₃,
-(CH₂)₄-CH(C₂H₅)-CH₃.

11. Composition selon l'une quelconque des revendications précédentes dans laquelle la quantité de monomères électrophile est comprise entre 0,1 et 80 % en poids du poids total de la composition.

12. Composition selon l'une quelconque des revendications précédentes, dans laquelle le ou les composés fluorescents sont choisis parmi les colorants fluorescents et les azurants optiques.

13. Composition selon la revendication 12 dans laquelle les colorants fluorescents présentent une longueur d'onde max d'émission dans la lumière visible orangée.

14. Composition selon la revendication 12 dans laquelle le ou les azurants optiques présentent une longueur d'onde max d'émission dans la lumière visible bleue.

15. Composition selon la revendication 12 dans laquelle le ou les colorants fluorescents sont choisis parmi les colorants de structures suivantes :

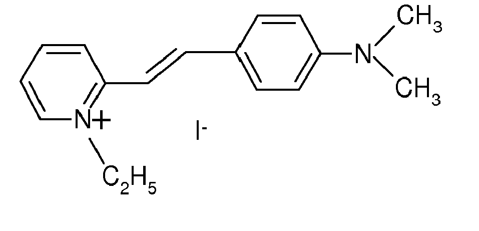

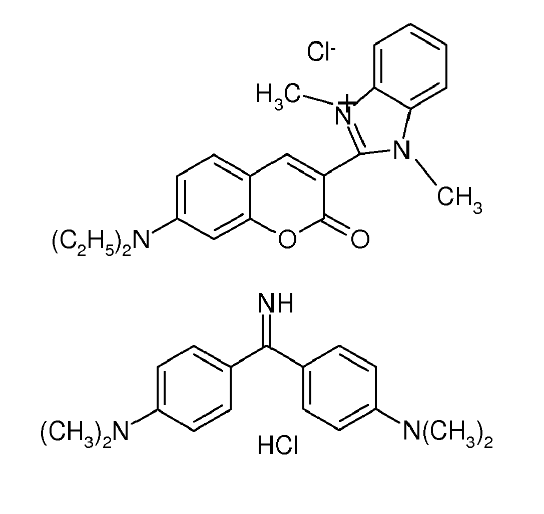

16. Composition selon la revendication 12, dans laquelle le ou les composés fluorescents sont choisis parmi les naphtalimides, les coumarines, les xanthènes, les rhodamines, les thioxanthènes, les naphtolactames, les azalactones, les méthiniques, les oxazines et thiazines, les distylbenzène, les distyrylbiphényles, les triazinylaminostilbènes, les stilbazolium et leurs dimères, leurs trimères et leurs tétramères, les bis(benzoxazole), les bis(benzimidazoles) cationiques ou non, les diphénylpyrrazolines anioniques ou non, les dicétopyrrolopyrrole, les dicyanopyrazines et les dérivés de toutes ces familles.

17. Composition selon l'une quelconque des revendications précédentes dans laquelle le ou les composés fluorescents sont chacun présents à des concentrations comprises entre 0,01 et 40 % du poids total de la composition.

18. Composition selon l'une quelconque des revendications précédentes anhydre

19. Composition selon l'une quelconque des revendications précédentes dans laquelle le solvant organique est choisi parmi les huiles organiques ; les silicones ; les huiles minérales ; les huiles végétales ; les alcanes en C₅-C₁₀, l'acétone, la méthyléthylcétone, les esters d'acides en C₁-C₂₀ liquides et d'alcools en C₁-C₈, le diméthoxyéthane, le diéthoxyéthane, les alcools gras liquides en C₁₀-C₃₀, les esters d'alcools gras en C₁₀-C₃₀ liquides ; l'huile de polybutène, l'isononanoate d'isononyle, le malate d'isostéaryle, le tétra-isostéarate de pentaérythrityle, le trimélate de tridécyle, le mélange cyclopentasiloxane (14,7% en poids)/polydiméthylsiloxane dihydroxylé en positions α et ω (85,3% en poids), ou leurs mélanges.

20. Composition selon l'une quelconque des revendications précédentes comprenant de plus un agent nucléophile.

21. Composition selon la revendication 20 dans laquelle l'agent nucléophile est l'eau.

22. Procédé de coloration des fibres kératiniques **caractérisé par le fait que** l'on applique sur les fibres kératiniques la composition telle que définie à l'une quelconque des revendications 1 à 20 en présence d'un agent nucléophile.

23. Procédé selon la revendication 22 dans lequel on applique la composition telle que définie à la revendication 20 ou 21.

24. Procédé selon la revendication 23 dans lequel une première étape qui consiste à appliquer une composition contenant le ou les composés fluorescents sur les fibres et une seconde étape qui consiste à appliquer une composition contenant le monomère électrophile, l'agent nucléophile étant présent dans la composition contenant le composé fluorescent ou dans une composition séparée.

25. Procédé selon la revendication 24 dans lequel la composition contenant le ou les composés fluorescents est une composition aqueuse et la composition qui contient le ou les monomères électrophiles est anhydre.

26. Procédé selon l'une quelconque des revendications 22 à 25 pour la coloration des fibres kératiniques présentant une hauteur de ton inférieur à 6, de préférence inférieure ou égale à 4 .

27. Kit de coloration comprenant une première composition qui contient au moins un composé fluorescent soluble, une seconde composition qui contient le ou les monomères électrophile tel que défini aux revendications 1 à 11 et éventuellement une troisième composition qui contient un agent nucléophile.

28. Kit selon la revendication 27 dans lequel la composition contenant le ou les composés fluorescents et le ou les monomères électrophiles sont présents dans une même composition anhydre.
